# EUROPÄISCHE PATENTSCHRIFT

(11) **EP 1 327 099 B1**
(45) Veröffentlichungstag und Bekanntmachung des Hinweises auf die Patenterteilung: **21.12.2005**
(21) Anmeldenummer: 01943245.9
(22) Anmeldetag: 11.04.2001
(51) Int. Cl.: F16M 11/04

(54) **FANGVORRICHTUNG FÜR EINE AUFHÄNGEEINRICHTUNG**
FASTENING DEVICE FOR A SUSPENSION SYSTEM
DISPOSITIF DE FIXATION POUR SYSTEME DE SUSPENSION

(30) Priorität: 19.10.2000 DE 10051898
(43) Veröffentlichungstag der Anmeldung: 16.07.2003
(73) Patentinhaber: Mavig GmbH, 81829 München (DE)
(72) Erfinder: KUHN, Peter, 81545 München (DE)
(74) Vertreter: Körfer, Thomas
(86) Internationale Anmeldenummer: PCT/EP2001/004164
(87) Internationale Veröffentlichungsnummer: WO 2002/033307

(56) Entgegenhaltungen:
- DE-U- 29 818 108
- GB-A- 2 200 491
- US-A- 5 937 073

## Beschreibung

Die Erfindung bezieht sich auf eine Fangvorrichtung zur Sicherung einer Aufhängeeinrichtung.

Aufhängeeinrichtungen der vorliegenden Art dienen dazu, Gebrauchsgegenstände in aufgehängten Gebrauchsstellungen im Bereich von Arbeitsplätzen bereitzuhalten. Bevorzugte Verwendungen für eine vorliegende Aufhängeeinrichtung sind medizinische Behandlungsplätze in ärztlichen Praxen und Kliniken sowie medizinisch-technische Arbeitsplätze in medizinischen Labors. Gebrauchsgegenstände, die mit einer Aufhängeeinrichtung bereitgestellt werden können, können z. B. Röntgenabschirmungen, Meßgeräte, Bildwiedergabegeräte (Monitore), Ablageteile für Arbeits- oder Behandlungswerkzeuge und dergleichen sein.

Aufgrund des teilweise hohen Gewichts der mittels der Aufhängeeinrichtung bereitgestellten Gebrauchsgegenstände und der häufigen Positions- und Höhenverstellung der zumeist mehrteiligen und langen Ausleger der Aufhängeeinrichtung kann es zu Ermüdungserscheinungen und nachfolgend Brüchen an Schwachstellen kommen. Dazu zählen insbesondere Gelenkverbindungen und Aufhängungspunkte. Um Unfälle und Verletzungen des sich im Bereich der Aufhängeeinrichtung aufhaltenden Personenkreises zu verhindern, muß daher sichergestellt sein, daß die Aufhängeeinrichtung bei Brüchen so gesichert ist, daß die abbrechenden Teile nicht bis zum Boden stürzen können.

Eine aus der DE 298 18 108 U1 bekannte Vorrichtung zur Befestigung einer Aufhängeeinrichtung an einem Träger umfaßt ein Sicherungselement, welches zusätzlich zu der Hauptbefestigung der Aufhängeeinrichtung beispielsweise an einem Träger oder einer Raumdecke verankert ist. Das Sicherungselement ist dabei mit einem ersten Ende an der Aufhängeeinrichtung und mit einem zweiten Ende am Träger oder an der Raumdecke fixiert. Es kann band-, ketten- oder seilförmig ausgebildet sein oder auch aus einer Teleskopstange bestehen.

Des weiteren ist aus der US 5,937,073 eine Sicherungsvorrichtung für eine beispielsweise an einer Raumdecke hängend angebrachte Halterung für einen Lautsprecher bekannt, wobei der Lautsprecher mittels eines Seils oder Kabels und eines Karabinerhakens mit der Halterung verbunden und damit gegen Herabstürzen gesichert ist.

Der Erfindung liegt die Aufgabe zugrunde, durch eine einfach aufgebaute und leicht montierbare Fangvorrichtung die Betriebssicherheit einer Aufhängeeinrichtung zu erhöhen und gleichzeitig Beschädigungen der durch die Aufhängeeinrichtung bereitgestellten Gebrauchsgegenstände zu verhindern.

Diese Aufgabe wird durch die Merkmale des Anspruchs 1 gelöst.

Bei der erfindungsgemäßen Fangvorrichtung weist ein Rückhalteteil eine Sicherungsschlinge auf, die in einen ein- oder mehrteilig ausgeführten Sicherungskörper der Fangvorrichtung einlegbar ist. Die Sicherungsschlinge ist so ausgelegt, daß sie entweder um einen Bolzen o. ä. im der Fangvorrichtung nächstliegenden Gelenk der Aufhängeeinrichtung oder im weiteren Verlauf der Tragarme schlingbar ist und somit einen mit dem Hauptteil der Fangvorrichtung verbundenen Gebrauchsgegenstand, der an der Aufhängeeinrichtung aufgehängt ist, in einfacher Weise gegen Herabstürzen sichert.

Vorteilhafterweise ist dabei sowohl der Gebrauchsgegenstand mit der Fangvorrichtung als auch die Fangvorrichtung mit der Aufhängeeinrichtung verbunden. Gleichzeitig sind alle drei Teile über einen Sicherungsstift, der durch das Hauptteil der Fangvorrichtung geschoben wird, verbunden, so daß die Fangvorrichtung sowohl bei Ermüdungsbrüchen der Aufhängeeinrichtung als auch bei einer Fehlfunktion oder einem Bruch des Verbindungselements des Gebrauchsgegenstandes die abgebrochenen Teile sichert.

Die Fangvorrichtung ist in einfacher Weise hergestellt und kann sowohl bei Neugeräten bei der Fertigung implementiert oder bei bereits installierten Aufhängeeinrichtungen nachgerüstet werden.

In den Unteransprüchen sind Merkmale enthalten, die die angestrebte Sicherheit weiter verbessern, zu einfachen und sicheren Befestigungsmaßnahmen führen, eine einfache und kostengünstig herstellbare Ausgestaltung ermöglichen und auch eine leichte und schnelle Montage gewährleisten.

Nachfolgend werden die Erfindung und weitere durch sie erzielbare Vorteile anhand bevorzugter Ausführungsbeispiele und einer Zeichnung näher beschrieben. Es zeigen:
- Fig. 1: eine erfindungsgemäße, in einem Gelenk einer Aufhängeeinrichtung montierte Fangvorrichtung in einer perspektivischen Gesamtdarstellung;
- Fig. 2A: eine perspektivische Darstellung des teilweise demontierten Gelenks mit einem ersten Ausführungsbeispiel der erfindungsgemäßen Fangvorrichtung;
- Fig. 2B: eine perspektivische Darstellung eines Verbindungselements zur Anbringung eines Gebrauchsgegenstands, welches mit der erfindungsgemäßen Fangvorrichtung kombinierbar ist;
- Fig. 3A: eine perspektivische Darstellung des ersten Ausführungsbeispiels der erfindungsgemäßen Fangvorrichtung;
- Fig. 3B: eine perspektivische Darstellung der einzelnen Bauteile des in Fig. 3A dargestellten ersten Ausführungsbeispiels der erfindungsgemäßen Fangvorrichtung;
- Fig. 4A bis 4C: Schnitte in verschiedenen Ebenen durch einen ersten Teil der erfindungsgemäßen Fangvorrichtung;
- Fig. 5A bis 5C: Schnitte in verschiedenen Ebenen durch einen zweiten Teil der erfindungsgemäßen Fangvorrichtung, und
- Fig. 6A bis 6B: zwei Ansichten eines zweiten Ausführungsbeispiels einer erfindungsgemäß ausgestalteten Fangvorrichtung.

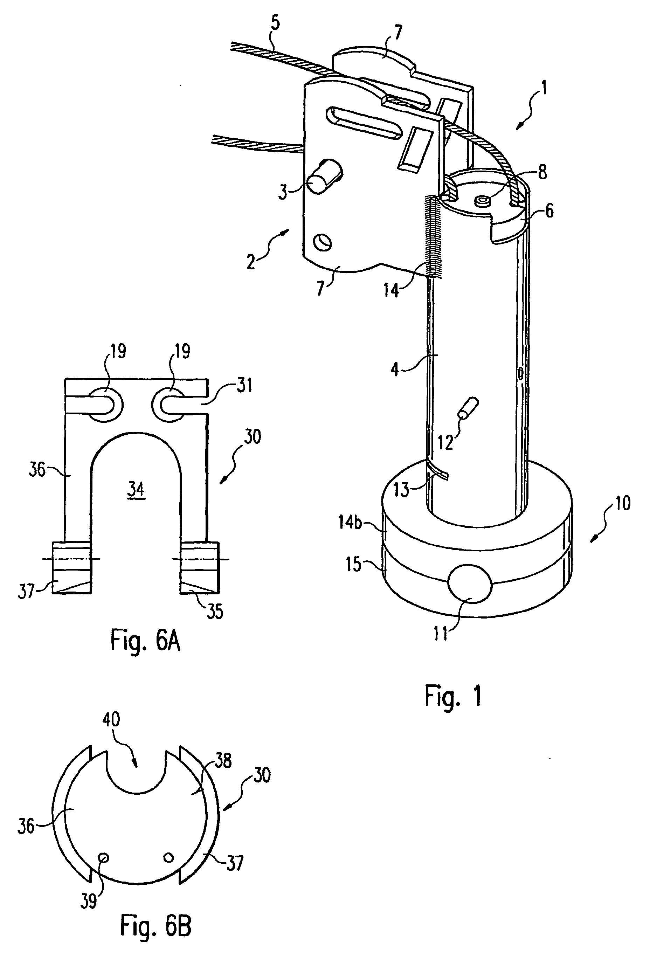

Fig. 1 zeigt in einer perspektivischen Darstellung ein erstes Ausführungsbeispiel einer erfindungsgemäß ausgestalteten Fangvorrichtung 1 in montierter Position in einer beispielhaften Aufhängeeinrichtung.

Die Aufhängeeinrichtung ist dabei nicht weiter dargestellt und wird in der Zeichnung durch eine Lagerzapfenaufnahme 2 repräsentiert. Die Lagerzapfenaufnahme 2 ist beispielsweise endständig an einem ein- oder mehrteiligen Tragarm der Aufhängeeinrichtung angebracht und bildet mittels einer mehrteiligen Bolzenverbindung ein Gelenk aus. Ein Bolzen 3 ist in Fig. 1 stellvertretend abgebildet.

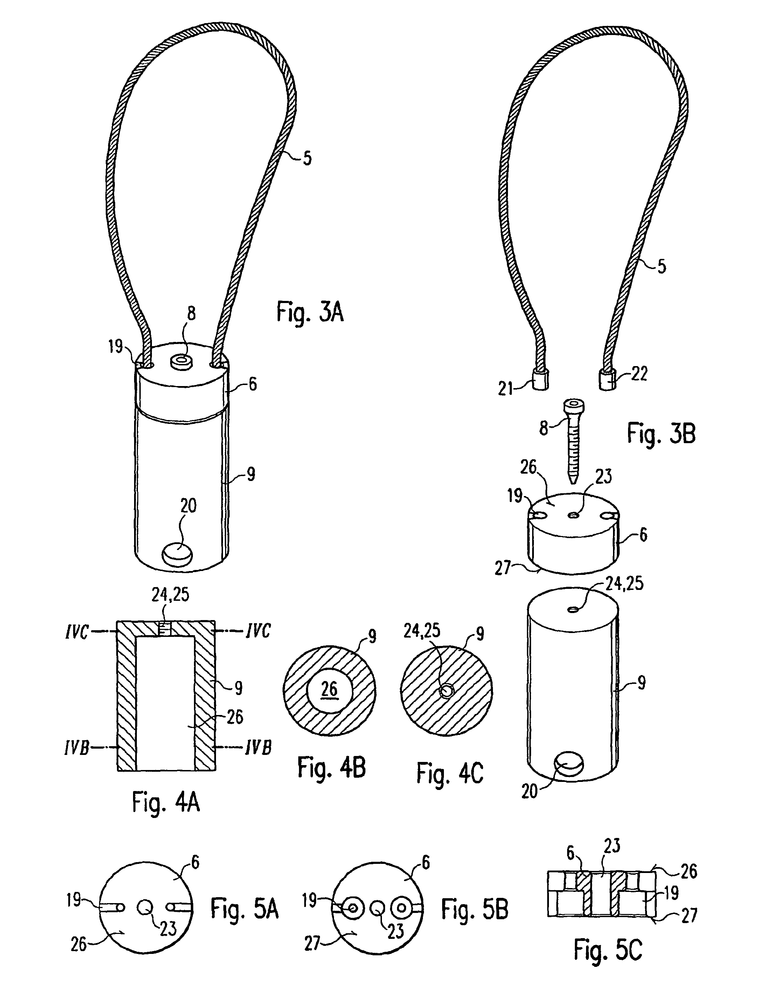

In einer Aufnahmehülse 4 der Lagerzapfenaufnahme 2 ist die Fangvorrichtung 1 eingeschoben. Eine Sicherungsschlinge 5, welche mit einem Rückhaltekörper 6 verbunden ist, ist zwischen Wangen 7 der Lagerzapfenaufnahme 2 so angeordnet, daß sie einen Bogen um den Bolzen 3 bildet. Die Sicherungsschlinge 5 kann dabei so dimensioniert sein, daß sie nur den Bolzen 3 umschlingt, sie kann aber auch so lang sein, daß sie sich beispielsweise teilweise oder ganz entlang des Tragarms erstreckt und im Bereich einer anderen Gelenkverbindung um einen Bolzen o. ä. geführt ist. Eine genauere Beschreibung der Fangvorrichtung ist den Fig. 3 bis 5 zu entnehmen.

Das Rückhalteteil 6 ist im ersten Ausführungsbeispiel mittels einer Schraube 8 mit einem Hauptteil 9 der Fangvorrichtung 1 lösbar verbunden, d. h. Rückhalteteil 6 und Hauptteil 9 sind bei diesem Ausführungsbeispiel zweiteilig ausgebildet.

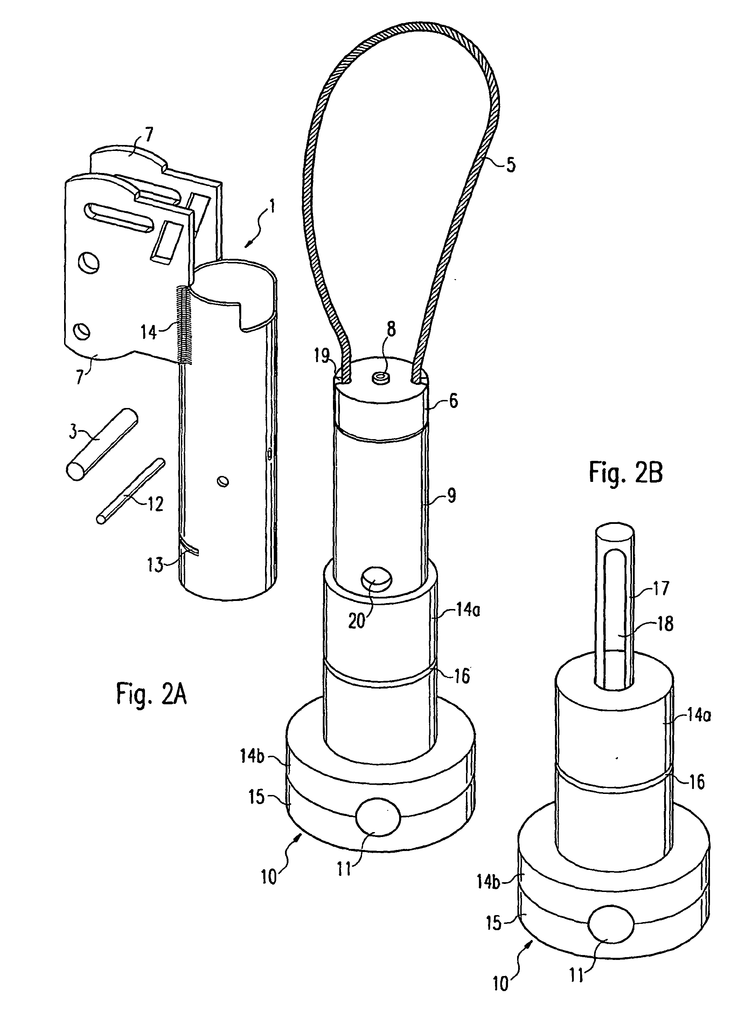

Die Aufnahmehülse 4 ist im fertig montierten Zustand über ein Verbindungselement 10 geschoben, welches in einer Ausnehmung 11 beispielsweise eine nicht weiter dargestellte Haltestange einer Röntgenabschirmung oder eines anderen Gebrauchsgegenstandes aufnehmen kann. Das Verbindungselement 10 ist in Fig. 2B näher dargestellt.

Ein Sicherungsstift 12, welcher durch die Aufnahmehülse 4, das Verbindungselement 10 und das Hauptteil 9 der Fangvorrichtung 1 geschoben wird, verbindet die genannten Bauteile miteinander. Ein Schlitz 13, der in der Aufnahmehülse 4 angebracht ist, kann eine weitere Sicherung, auf die hier nicht weiter eingegangen wird, aufnehmen.

Durch die beschriebene Anordnung der erfindungsgemäß ausgestalteten Fangvorrichtung 1 kann somit sichergestellt werden, daß sowohl ein Bruch einer die Wangen 7 mit der Aufnahmehülse 4 verbindenden Schweißnaht 14 als auch ein Riß der Aufnahmehülse 4 an einer Schwachstelle, beispielsweise im Bereich des Schlitzes 13,- nicht zum Hinunterstürzen eines an der Aufhängeeinrichtung aufgehängten Gebrauchsgegenstandes führt. Der Sturz wird durch die um den Bolzen 3 geschlungene Sicherungsschlinge 5 abgefangen, wobei der Fallweg z. B. nur etwa 0,1 m beträgt. Dadurch kann zusätzlich bewirkt werden, daß der Bruch der Aufnahmehülse 4 nicht unbemerkt bleibt, wie es beispielsweise bei einer straff um den Bolzen 3 gelegten Sicherungsschlinge 5 der Fall wäre, und eine Reparatur veranlaßt wird.

Fig. 2A und 2B zeigen in einer perspektivischen Darstellung die teilweise demontierte Anordnung aus Fig. 1. Übereinstimmende Bauteile sind mit übereinstimmenden Bezugszeichen versehen.

In Fig. 2A sind dazu der Stift 12 und der Bolzen 3 entfernt und die Aufnahmehülse 4 abgezogen worden. Die Fangvorrichtung 1 sitzt auf dem Verbindungselement 10 auf, welches, wie in Fig. 2B dargestellt, aus einem einstückig ausgebildeten, zylindrisch stufenförmig geformten ersten Teil 14 und einem ebenfalls zylindrisch oder scheibenförmig geformten zweiten Teil 15 besteht. Zwischen dem ersten Teil 14 und dem zweiten Teil 15 ist die Ausnehmung 11 ausgebildet, die beispielsweise die Querstange einer Röntgenabschirmung aufnimmt. Eine im ersten Teil 14 ausgebildete Nut 16 korrespondiert mit der oben erwähnten, nicht näher beschriebenen Zusatzsicherung.

Der erste Teil 14 des Verbindungselements 10 weist einen Sicherungsbolzen 17 auf, der in dem hohlzylindrischen ersten Teil 14 geführt ist und aus diesem herausragt. In dem Sicherungsbolzen 17 ist ein Langloch 18 ausgebildet, welches in montiertem Zustand der Aufhängeeinrichtung von dem Stift 12 durchgriffen wird. Im montierten Zustand steckt das ebenfalls hohlzylindrische Hauptteil 9 der Fangvorrichtung 1 auf dem Sicherungsbolzen 17 des Verbindungselements 10.

Fig. 3A zeigt in- einer perspektivischen Darstellung die erfindungsgemäße Fangvorrichtung 1 ohne die sonstigen Bauteile.

Wie bereits weiter oben erwähnt, ist das Hauptteil 9 der Fangvorrichtung hohlzylindrisch ausgebildet. Er ist mittels einer Schraube 8 oder eines ähnlichen Befestigungsgegenstandes mit dem Rückhalteteil 6 verbunden, das wiederum die Sicherungsschlinge 5 fixiert. Die Sicherungsschlinge 5 ist dabei durch Schlitzbohrungen 19 im Rückhalteteil 6 geführt. Eine Bohrung 20 im Hauptteil 9 der Fangvorrichtung 1 dient der Arretierung durch den Sicherungsstift 12 mit den übrigen Bauteilen.

Fig. 3B zeigt in einer perspektivischen Darstellung die einzelnen Bauteile der Fangvorrichtung 1.

Die Sicherungsschlinge 5 besteht aus einem Seil, einem Band, einer Kette oder ähnlichem und ist im vorliegenden ersten Ausführungsbeispiel als Stahlseil ausgeführt. Dies hat den Vorteil hoher Bruchfestigkeit, großer Flexibilität und geringen Durchmessers. Die Sicherungsschlinge 5 weist dabei an ihren Enden 21 je eine Verbreiterung 22 auf, die beispielsweise aus aufgesteckten und anschließend verpreßten Hülsen bestehen. Die Enden 21 der Sicherungsschlinge 5 mit den Verbreiterungen 22 werden dann in die Schlitzbohrungen 19 des Rückhalteteils 6 eingelegt und untergreifen diesen durch die spezielle Form der Schlitzbohrungen 19.

Das Rückhalteteil 6 weist neben den Schlitzbohrungen 19 für die Sicherungsschlinge 5 eine Bohrung 23 auf, die wahlweise glatt oder mit einem Gewinde versehen sein kann und von der Schraube 8 durchgriffen wird. Die Bohrung 23 korrespondiert mit einer Bohrung 24 des Hauptkörpers 9, welche mit einem zur Schraube 8 passenden Gewinde 25 versehen ist, so daß das Hauptteil 9 und das Rückhalteteil 6 in diesem Ausführungsbeispiel lösbar verbindbar sind.

Zur Montage der Fangvorrichtung 1 wird zunächst die Sicherungsschlinge 5 um den Bolzen 3 gelegt, dann die Verbreiterungen 22 der Enden 21 der Sicherungsschlinge 5 in die Schlitzbohrungen 19 eingelegt und schließlich durch Eindrehen der Schraube 8 das Rückhalteteil 6 mit dem Hauptteil 9 verbunden. Danach kann die Fangvorrichtung in die Aufnahmehülse 4 eingeschoben, auf den Sicherungsbolzen 17 des Verbindungselements 10 aufgesetzt und durch den Sicherungsstift 12 mit den genannten Bauteilen verbunden werden.

Fig. 4A bis 4C zeigen zur Verdeutlichung drei schematische Schnitte durch das Hauptteil 9 der erfindungsgemäßen Fangvorrichtung 1. Der in Fig. 4A dargestellte erste Schnitt verläuft dabei in axialer Richtung.

Das Hauptteil 9 der Fangvorrichtung 1 ist hohlzylindrisch ausgeführt und weist eine Ausnehmung 26 auf. Die Ausnehmung 26 setzt sich in der Bohrung 24 mit dem Gewinde 25 fort. Durch die einfache Form des Hauptteils 9 der Fangvorrichtung 1 ist eine einfache und kostengünstige Herstellung, beispielsweise durch Drehen, möglich.

Fig. 4B zeigt einen Schnitt entlang der Linie IVB-IVB in Fig. 4A, Fig. 4C einen Schnitt entlang der Linie IVC-IVC. Beide Schnitte verdeutlichen nochmals die einfache Form des Hauptteils 9, welche nicht auf runde Querschnitte beschränkt ist. Ebenso sind elliptische oder mehrkantige Querschnitte denkbar.

Fig. 5A bis 5C stellt verschiedene Ansichten sowie einen Längsschnitt durch das Rückhalteteil 6 dar. Dabei zeigt Fig. 5A eine Aufsicht auf eine dem Hauptteil 9 abgewandte Stirnseite 26, Fig. 5B eine Aufsicht auf eine dem Hauptteil 9 zugewandte Stirnseite 27 und Fig. 5C einen Schnitt entlang der Schlitzbohrungen 19 durch das Rückhalteteil 6.

Wie aus den Fig. 5A und 5B ersichtlich, sind die Schlitzbohrungen 19, die sich durch das Rückhalteteil 6 erstrecken, in ihrem Durchmesser variabel. Die Schlitzbohrungen 19 sind dabei so geformt, daß die Verbreiterungen 22 an den Enden 21 der Sicherungsschlinge 5 das Rückhalteteil 6 durch- und gleichzeitig untergreifen, so daß sie in axialer Richtung fest arretiert sind und dementsprechend ihrer Sicherungsfunktion nachkommen, während sie zur Montage in radialer Richtung aus den Schlitzbohrungen 19 entfernt werden können. Die Verbreiterungen 22 sind dabei zweckmäßigerweise so dimensioniert, daß sie mit der dem Hauptteil 9 zugewandten Stirnseite 27 bündig ohne Überstand abschließen.

Fig. 5C verdeutlicht nochmals die zweiteilige Form der Schlitzbohrungen 19, deren Durchmesser sich von der dem Hauptteil 9 zugewandten Stirnseite 27 zur gegenüberliegenden Stirnseite 26 des Rückhalteteils 6 stufenförmig verkleinern und somit eine sehr zugfeste, aber mechanisch einfache Arretierung der Sicherungsschlinge 5 in dem Rückhalteteils 6 ermöglichen.

Fig. 6A und 6B zeigen zwei Ansichten eines zweiten Ausführungsbeispiels einer erfindungsgemäß ausgestalteten Fangvorrichtung 1, bei der das Hauptteil 9 und das Rückhalteteil 6 einstückig miteinander ausgebildet sind. Im Folgenden wird das Gesamtbauteil mit dem Begriff Sicherungskörper 30 bezeichnet.

Im Gegensatz zu dem oben beschriebenen ersten Ausführungsbeispiel einer erfindungsgemäß ausgestalteten Fangvorrichtung 1 weist das im folgenden dargestellte zweite Ausführungsbeispiel einen einteiligen Sicherungskörper 30 auf, an welchem eine umlaufende radiale Nut 31 ausgebildet ist.

Die bereits weiter oben beschriebene Sicherungsschlinge 5 kann in der gleichen Weise wie im vorigen Ausführungsbeispiel als Ketten-, Band- oder Drahtschlinge 5 ausgeführt sein. Sie weist an ihren Enden 21 Verbreiterungen 22 auf, welche beispielsweise aus aufgesteckten und dann verquetschten Hülsen bestehen.

Fig. 6A zeigt eine seitliche Ansicht des einteiligen Sicherungskörpers 30. Die umfängliche radiale Nut 31 weist Öffnungen 19 auf, in die die in Fig. 6A nicht dargestellte Sicherungsschlinge 5 einrastbar ist.

Zur Aufnahme des Sicherungsbolzens 17 des Verbindungselements 10 weist der Sicherungskörper 30 eine U-förmige Ausnehmung 34 auf. Zur besseren Anpassung an die Form des Verbindungselements 10 ist zusätzlich eine innenseitig an der Ausnehmung 34 ausgebildete Fase 35 vorgesehen.

Der Sicherungskörper 30 ist zweistufig ausgeführt, wobei in einer ersten Stufe 36 die umlaufende Nut 31 und in einer zweiten Stufe 37, deren Durchmesser den Durchmesser der ersten Stufe 36 übersteigt, die Bohrung 20 ausgebildet ist, die der Arretierung durch den in Fig. 6A nicht weiter dargestellten Sicherungsstift 12 mit den übrigen Bauteilen dient.

Fig. 6B zeigt eine Aufsicht von oben auf den Sicherungskörper 30 der erfindungsgemäß ausgestalteten Fangvorrichtung 1. Die Verbreiterung der zweiten Stufe 37, die einen größeren Durchmesser als die ersten Stufe 36 aufweist, ist deutlich erkennbar.

In einer Stirnseite 38 der ersten Stufe 36 sind Bohrungen 39 vorgesehen, die so angeordnet sind, daß in die Bohrungen 39 eingeschlagene, hier nicht dargestellte Sicherungsstifte die Position der Verbreiterungen 22 der Enden 21 der Sicherungsschlinge 5 fixieren und ein Herausrutschen aus der umfänglichen Nut 31 verhindern.

Weiterhin ist eine axiale Ausnehmung 40 vorgesehen, die sich randständig durch die erste Stufe 36 und die zweite stufe 37 des Sicherungskörpers 30 erstreckt. Die axiale Ausnehmung 40 ist insbesondere zur Aufnahme eines Kabels geeignet, welches dem an der Aufhängeeinrichtung angeordneten Gebrauchsgegenstand als elektrische Versorgung dient.

Zur Rücksicherung des aufgehängten Gebrauchsgegenstandes wird die Sicherungsschlinge 5, wie bereits weiter oben beschrieben, um den Bolzen 3 der Lagerzapfenaufnahme 2 der Aufhängeeinrichtung gelegt und mit ihren Enden 21 in die radiale umfängliche Nut 31 des Sicherungskörpers 30 eingelegt. Dabei rasten die Verbreiterungen 22 der Enden 21 in die Öffnungen 19 des Sicherungskörpers 30 ein. Dann werden in die Bohrungen 39 Sicherungsstifte eingeschlagen, welche so positioniert sind, daß die Verbreiterungen 22 der Enden 21 der Sicherungsschlinge 5 in den Öffnungen 19 der umfänglichen Nut 31 des Sicherungskörpers 30 ortsfest fixiert sind.

Durch die radial angeordnete Sicherungsschlinge 5 ist eine platzsparende Rücksicherung möglich, die nur in Umfangsrichtung belastet wird und somit einen hohen Sicherheitsgrad gewährleistet.

## Patentansprüche

1. Fangvorrichtung (1) zur Sicherung von an einer Aufhängeeinrichtung aufgehängten Gebrauchsgegenständen gegen Herabstürzen, mit einer Sicherungsschlinge (5), die zur Verbindung mit der Aufhängeeinrichtung um einen Bolzen (3) der Aufhängeeinrichtung schlingbar ist, wobei die Sicherungsschlinge (5) Verbreiterungen (22) an ihren Enden (21) aufweist, die in einem Rückhalteteil (6) ausgebildete Öffnungen (19) durchgreifen, wobei die Öffnungen (19) im Rückhalteteil (6) kleiner sind als die Verbreiterungen (22) der Sicherungsschlinge (5), und mit einem Hauptteil (9), der mit einem Sicherungsbolzen (17) eines Verbindungselements (10), welches an dem Gebrauchsgegenstand fixiert ist, durch einen Stift (12) verbindbar ist, wobei das Hauptteil (9) mit dem Rückhalteteil (6) lösbar verbindbar ist.

2. Fangvorrichtung nach Anspruch 1,
**dadurch gekennzeichnet,**
**daß** das Hauptteil (9) hohlzylindrisch ausgebildet ist.

3. Fangvorrichtung nach Anspruch 1 oder 2,
**dadurch gekennzeichnet,**
**daß** das Hauptteil (9) mit dem Rückhalteteil (6) mittels eines in einer Bohrung (24) des Hauptteils (9) angebrachten Gewindes (25) und einer Schraube (8) verschraubbar sind.

4. Fangvorrichtung nach einem der Ansprüche 1 bis 3,
**dadurch gekennzeichnet,**
**daß** sich der Durchmesser der in dem Rückhalteteil (6) ausgebildeten Öffnungen (19) von einer dem Hauptteil (9) zugewandten Stirnseite (27) des Rückhalteteils (6) zu einer dazu entgegengesetzten Stirnseite (26) stufenförmig verringert.

5. Fangvorrichtung nach einem der Ansprüche 1 bis 4,
**dadurch gekennzeichnet,**
**daß** sich die Öffnungen (19) in axialer Richtung des Rückhalteteils (9)erstrecken.

6. Fangvorrichtung nach einem der Ansprüche 1 bis 5,
**dadurch gekennzeichnet,**
**daß** die Sicherungsschlinge (5) in Form einer Bandschlinge, einer Kettenschlinge oder einer Drahtschlinge ausgebildet ist.

7. Fangvorrichtung nach einem der Ansprüche 1 bis 6,
**dadurch gekennzeichnet,**
**daß** die Verbreiterungen (22) in die Öffnungen (19) des Rückhalteteils (6) einrastbar sind.

8. Fangvorrichtung nach Anspruch 1,
**dadurch gekennzeichnet,**
**daß** das Hauptteil (6) und das Rückhalteteil (9) einstückig zu einem Sicherungskörper (30) ausgebildet sind.

9. Fangvorrichtung nach Anspruch 8,
**dadurch gekennzeichnet,**
**daß** der Sicherungskörper (30) eine umfängliche, radiale Nut (31) aufweist.

10. Fangvorrichtung nach Anspruch 8 oder 9,
**dadurch gekennzeichnet,**
**daß** die umfängliche Nut (31) Öffnungen (19) aufweist.

11. Fangvorrichtung nach Anspruch 10,
**dadurch gekennzeichnet,**
**daß** die Verbreiterungen (22) an den Enden (21) der Sicherungsschlinge (5) in die Öffnungen (19) der umfänglichen Nut (31) einrastbar sind.

12. Fangvorrichtung nach Anspruch 10 oder 11,
**dadurch gekennzeichnet,**
**daß** die Enden (21) der Sicherungsschlinge (5) in den Öffnungen (19) mittels Sicherungsstiften arretierbar sind.

13. Fangvorrichtung nach einem der Ansprüche 1 bis 12,
**dadurch gekennzeichnet,**
**daß** die Fangvorrichtung (1) eine randständige axiale Ausnehmung (40) aufweist.

## Claims

1. A catching device (1) for securing utensils suspended on a suspension device against falling, having a securing loop (5), which can be looped around a bolt (3) of the suspension device for connection to the suspension device, the securing loop (5) having widened parts (22) at its ends (21), which reach through openings (19) made in a retention part (6), the openings (19) in the retention part (6) being smaller than the widened parts (22) of the securing loop (5), and having a main part (9) which can be connected by a pin (12) to a securing bolt (17) of a connecting element (10), which is fixed to the utensil, the main part (9) being detachably connected to the retention part (6).

2. A catching device according to Claim 1,
**characterised in that** the main part (9) is of a hollow cylindrical construction.

3. A catching device according to Claim 1 or 2,
**characterised in that**
the main part (9) can be screwed to the retention part 6) by means of a thread (25) incorporated in a bore (24) of the main part (9) and a screw (8).

4. A catching device according to one of Claims 1 to 3,
**characterised in that**
the diameter of the openings (19) made in the retention part (6) decreases in stepped manner from an end face (27), facing the main part (9), of the retention part (6) to an opposing end face (26).

5. A catching device according to one of Claims 1 to 4,
**characterised in that**
the openings (19) extend in the axial direction of the retention part (9).

6. A catching device according to one of Claims 1 to 5,
**characterised in that**
the securing loop (5) is constructed in the form of a strap, a chain loop or a wire loop.

7. A catching device according to one of Claims 1 to 6,
**characterised in that**
the widened parts (22) can be latched into the openings (19) in the retention part (6).

8. A catching device according to Claim 1,
**characterised in that**
the main part (6) and the retention part (9) are constructed in one piece to form a securing body (30).

9. A catching device according to Claim 8,
**characterised in that**
the securing body (30) has a circumferential radial groove (31).

10. A catching device according to Claim 8 or 9,
**characterised in that**
the circumferential groove (31) has openings (19).

11. A catching device according to Claim 10,
**characterised in that**
the widened parts (22) at the ends (21) of the securing loop (5) can be latched into the openings (19) of the circumferential groove (31).

12. A catching device according to Claim 10 or 11,
**characterised in that**
the ends (21) of the securing loop (5) can be locked in the openings (19) by means of securing pins.

13. A catching device according to one of Claims 1 to 12,
**characterised in that**
the catching device (1) has an axial cutout (40) along the edge.

## Revendications

1. Dispositif de fixation (1) pour la protection contre la chute d'objets usuels suspendus sur une installation de suspension, avec une boucle de fixation (5) qui est nouable pour la liaison avec l'installation de suspension autour d'un axe (3) de l'installation de suspension, la boucle de fixation (5) comprenant des évasements (22) à ses extrémités (21) qui s'engagent dans des ouvertures (19) configurées dans une pièce de retenue (6), les ouvertures (19) de la pièce de retenue (6) étant plus petites que les évasements (22) de la boucle de fixation (5), et avec une pièce principale (9) qui est reliable par une broche (12) à un axe de fixation (17) d'un élément de liaison (10) qui est fixé à l'objet usuel, la partie principale (9) étant reliable de façon amovible à la pièce de retenue (6).

2. Dispositif de fixation selon la revendication 1
**caractérisé en ce que**,
la pièce principale (9) est une pièce cylindrique creuse.

3. Dispositif de fixation selon la revendication 1 ou 2,
**caractérisé en ce que**,
la pièce principale (9) avec la pièce de retenue (6) peuvent être vissées au moyen d'un filetage (25) prévu dans un alésage (24) de la pièce principale (9) et d'une vis (8).

4. Dispositif de fixation selon une des revendications 1 à 3,
**caractérisé en ce que**,
le diamètre des ouvertures (19) formées dans la pièce de retenue (6) diminue progressivement depuis une face frontale (27) de la pièce de retenue (6) orientée vers la pièce principale (9) vers une face frontale (26) opposée à celle-ci.

5. Dispositif de fixation selon une des revendications 1 à 4,
**caractérisé en ce que**,
les ouvertures (19) s'étendent dans le sens axial de la pièce de retenue (6).

6. Dispositif de fixation selon une des revendications 1 à 5,
**caractérisé en ce que**,
la boucle de fixation (5) a la forme d'une boucle en bande, en chaîne ou en fil de fer.

7. Dispositif de fixation selon une des revendications 1 à 6,
**caractérisé en ce que**,
les évasements (22) sont enclenchables dans les ouvertures (19) de la pièce de retenue (6).

8. Dispositif de fixation selon la revendication 1,
**caractérisé en ce que**,
la pièce principale (9) et la pièce de retenue (6) sont configurées d'un seul tenant en un corps de fixation (30).

9. Dispositif de fixation selon la revendication 8,
**caractérisé en ce que**,
le corps de fixation (30) comprend une rainure (31) radiale périphérique.

10. Dispositif de fixation selon la revendication 8 ou 9,
**caractérisé en ce que**,
la rainure périphérique (31) comprend des ouvertures (19).

11. Dispositif de fixation selon la revendication 10,
**caractérisé en ce que**,
les évasements (22) aux extrémités (21) de la boucle de fixation (5) sont enclenchables dans les ouvertures (19) de la rainure périphérique (31).

12. Dispositif de fixation selon la revendication 10 ou 11,
**caractérisé en ce que**,
les extrémités (21) de la boucle de fixation (5) sont blocables dans les ouvertures (19) au moyen de broches de fixation.

13. Dispositif de fixation selon une des revendications 1 à 12,
**caractérisé en ce que**,
le dispositif de fixation (1) comprend un évidement (40) axial sur le bord.
